# EUROPÄISCHE PATENTANMELDUNG

(11) **EP 1 639 969 A1**
(43) Veröffentlichungstag der Anmeldung: **29.03.2006**
(21) Anmeldenummer: 04022671.4
(22) Anmeldetag: 23.09.2004
(51) Int. Cl.: A61F 2/44

(54) **Intravertebrale Prothese zur Überbrückung eines Wirbelkörpers**

(71) Anmelder: Cervitech, Inc., Rockaway, NJ 07866 (US)
(72) Erfinder: Keller, Arnold, 23863 Kayhude (DE)
(74) Vertreter: Glawe, Delfs, Moll

(57) **Zusammenfassung**

Prothese für den Teilersatz eines Wirbelkörpers mit einer oberen Anschlußplatte (5) zur Verbindung mit einem oberen Wirbelkörper (1), einer unteren Anschlußplatte (6) zur Verbindung mit einem unteren Wirbelkörper (2) und einem die obere und die untere Anschlußplatte (5, 6) miteinander verbindenden Überbrückungsteil (7) zum Überbrücken mindestens eines zwischen dem oberen und dem unteren Wirbelkörper (1, 2) befindlichen, teilweise zu ersetzenden Wirbelkörpers (3). Der Überbrückungsteil (7) findet in einer Ausnehmung des Wirbelkörpers (3) Platz. Um darin gesichert zu werden, weist er seitliche Verankerungsvorsprünge (9) auf, die in das beiderseits des Überbrückungsteils (7) befindliche Knochenmaterial eindringen. Sein Querschnitt verjüngt sich nach hinten vorzugsweise trapezförmig.

## Beschreibung

Die Erfindung betrifft eine Prothese nach dem Oberbegriff des Anspruchs 1. Diesem liegt eine bekannte Prothese zugrunde (DE-A-4109941, Fig. 2), die eine obere Anschlußplatte zur Verbindung mit einem oberen Wirbelkörper, eine untere Anschlußplatte zur Verbindung mit einem unteren Wirbelkörper und einen die obere und die untere Anschlußplatte verbindenden Überbrückungsteil zum Überbrücken mindestens eines zwischen dem oberen und unteren Wirbelkörper befindlichen Wirbelkörpers umfaßt, dessen Funktion durch die Prothese ersetzt werden soll. Die Querschnittsgröße des Überbrückungsteils ist wesentlich geringer als diejenige dieses Wirbelkörpers. Wenn er mehr oder minder vollständig erhalten ist, soll der Überbrückungsteil in ihn eingesetzt werden, so daß er vollständig darin eingebettet ist. Wie dies operativ geschehen könnte, ist unklar. Wenn er lediglich auf der Wirbelbogenseite noch einigermaßen vollständig ist, wird auf seiner Vorderseite eine Ausnehmung geschaffen, in die der Überbrückungsteil eingesetzt wird. Zur festen Verbindung mit dem Wirbelkörper weist der Überbrückungsteil seitlich vorspringende Stege auf, die ein Langloch zur Aufnahme einer Befestigungsschraube enthalten. Die Befestigung der Prothese am Wirbelkörper bestimmt zusätzlich zu den Facettengelenken dessen Stellung zu den benachbarten Wirbelkörpern. Nur wenn die zur Anlage der Befestigungsstege bestimmten Flächen des Wirbelkörpers so bearbeitet sind, daß dieser nach der Verbindung mit den Stegen seine durch die Facettengelenke vorgegebene natürliche Stellung beibehalten kann, besteht Aussicht auf einen beschwerdefreien Sitz der Prothese. Eine solche genaue Bearbeitung ist schwer zu erreichen. Außerdem hat sich gezeigt, daß eine Schraubbefestigung nicht sicher genug ist.

Bekannt ist auch eine Wirbelsäulenprothese (EP-A-1417940), bei welcher der Überbrückungsteil in Seitenansicht U-förmig ausgebildet ist, um mit seinen Schenkeln den zu ersetzenden Wirbelkörper unter- und oberseitig zu umfassen. Der Steg liegt auf der Vorderseite des Wirbelkörpers und wird mit diesem verschraubt. Dies setzt eine passende Bearbeitung des Wirbelkörpers auf seiner Ober-, Unter- und Vorderseite voraus, was insbesondere bei geschädigtem Wirbelkörper schwierig sein kann. Ferner sind Wirbelsäulenprothesen bekannt, bei denen der Überbrückungsteil den Wirbelkörper vollständig ersetzt (EP-A-567424, WO 0103614, DE-U-20115281, US-A-5895428). Dies hat den Nachteil, daß eine stützende Verbindung zwischen den verbleibenden Teilen des Wirbels und dem Überbrückungsteil kaum möglich ist.

Der Erfindung liegt daher die Aufgabe zugrunde, eine Wirbelsäulenprothese der eingangs genannten Art zu schaffen, die auch dann verwendet werden kann, wenn der zu ersetzende Wirbelkörper vollständig oder zu einem wesentlichen Teil erhalten ist. Sie strebt ferner an, daß eine vergleichsweise einfache Operationstechnik eine gut stützende Verbindung zwischen dem Implantat und dem zu ersetzenden Wirbelkörper verspricht.

Dies gelingt durch die Merkmale des Anspruchs 1. Es ist vergleichsweise leicht, von der Vorderseite her in dem Wirbelkörper eine Ausnehmung zu schaffen, die zu der Gestalt des Überbrückungsteils paßt und diesen im wesentlichen vollständig aufnimmt. Der Überbrückungsteil wird entsprechend schmaler als der Wirbelkörper ausgebildet. Durch den gegenseitigen Formschluß zwischen dem Überbrückungsteil und der Ausnehmung stützen sich der Überbrückungsteil und der Wirbelkörper gegenseitig. Der Überbrückungsteil kann der Ausnehmung auch nicht entweichen, weil seine seitlichen Vorsprünge ihn in der Ausnehmung festhalten.

Die gegenseitige Stützwirkung und die Sicherung durch die seitlichen Vorsprünge des Überbrückungsteils sind um so besser, je genauer die Ausnehmung der Form des Überbrückungsteils angepaßt ist. Dies gelingt am leichtesten dann, wenn die Querschnittsform der Ausnehmung rechteckig oder trapezförmig ist. Dies hat auch den Vorteil, daß die Seitenflächen des Überbrückungsteils groß sind und es dadurch erleichtern, eine Mehrzahl von Verankerungsvorsprüngen unterzubringen. Dies gilt insbesondere dann, wenn diese starr an dem Überbrückungsteil angeordnet sind, beispielsweise in der Form einer Vielzahl von kleinen Spitzen. Diese sind zweckmäßigerweise so gestaltet, daß sie beim Eindrücken des Überbrückungsteils in die Ausnehmung durch elastische oder plastische Nachgiebigkeit des Knochengewebes ihren Weg zu ihrem Verankerungsplatz finden. Sie können auch selbstschneidend ausgebildet sein. Eine andere Ausführungsmöglichkeit besteht darin, daß die Vorsprünge als Rauheit sehr klein ausgebildet sind. Dies genügt in der Regel um eine Anfangsfestigkeit der Implantat-Knochen-Verbindung zu schaffen und nach kurzer Zeit durch in die Rauhigkeit einwachsendes Knochengewebe eine dauerhafte Verbindung zu ermöglichen. Die Vorsprünge können widerhakenartig ausgeführt sein, um der Bewegung des Im-plantats in die Ausnehmung des Knochens geringen, aber der Entfernung aus der Ausnehmung größeren Widerstand entgegenzusetzen.

Der Überbrückungsteil kann mindestens auf den Seitenflächen Öffnungen oder Poren für die Aufnahme von Knochengewebe aufweisen. Diese können schon vor der Implantation mit Knochenspänen gefüllt werden. Im Laufe der Zeit wächst lebendes Knochengewebe ein, um einen festen Verbund zwischen dem Implantat und den Knochen zu ermöglichen.

Besondere Vorteile hat die Erfindung in der Anwendung bei der Halswirbelsäule.

Die Erfindung wird im folgenden näher unter Bezugnahme auf die Zeichnung erläutert, die vorteilhafte Ausführungsbeispiele veranschaulicht. Es zeigen:
- Fig. 1: einen Längsschnitt eines Ausführungsbeispiels in der Medianebene,
- Fig. 2: eine Frontalansicht desselben Implantats und
- Fig. 3: eine perspektivische Darstellung des Überbrückungsteils.

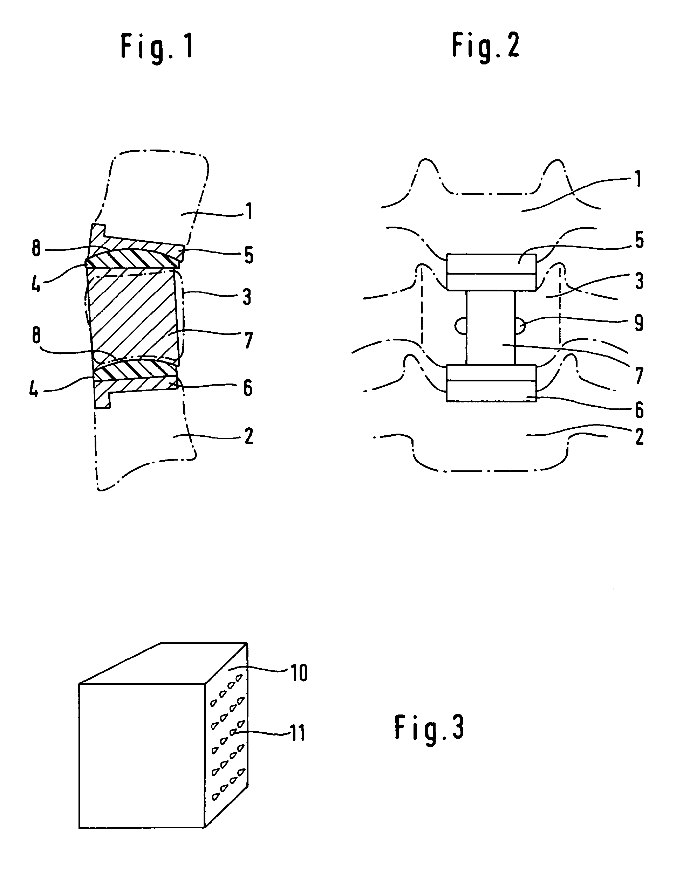

Fig. 2 zeigt einen oberen Wirbelkörper 1 und einen unteren Wirbelkörper 2, zwischen denen sich der zu ersetzende Wirbelkörper 3 befindet. Zwischen dem oberen und dem unteren Wirbelkörper ist eine Wirbelsäulenprothese eingesetzt. Sie umfaßt eine obere Anschlußplatte 5, die mit dem oberen Wirbelkörper 1 verbunden ist, eine untere Anschlußplatte 6, die mit dem unteren Wirbelkörper 2 verbunden ist, und einen Überbrückungsteil 7, der die Platten 5 und 6 verbindet.

Zwischen den Anschlußplatten 5 und 6 und dem Überbrückungsteil 7 befindet sich jeweils ein Gelenk (beispielsweise gemäß EP-A-560140) mit einer Gelenkfläche 8. Diese wird im oberen Gelenk einerseits von der Unterseite der Anschlußplatte 5 und andererseits von einem Gelenkteil 4 gebildet, der mit dem Überbrückungsteil 7 in nicht dargestellter Weise verbunden ist. Im unteren Gelenk wird sie einerseits von der Unterseite des Überbrückungsteils 7 und andererseits von dem Gelenkteil 4 gebildet, der mit der unteren Anschlußplatte 6 in nicht dargestellter Weise verbunden ist. Statt eines Gelenks mit sphärischer Gelenkfläche kann auch ein anderer Gelenktyp verwendet werden, beispielsweise ein solcher mit flexiblem Kissen (DE-U-20115281) oder mit Biegefeder (DE-A-4109941). Falls der obere Wirbelkörper 1 und der untere Wirbelkörper 2 starr verbunden werden sollen, können die Gelenke auch gänzlich entfallen. Schließlich ist es möglich, nur ein Gelenk zwischen der oberen Anschlußplatte 5 und dem Überbrückungsteil 7 oder zwischen der unteren Anschlußplatte 6 und dem Überbrückungsteil 7 zu verwenden.

Während die Anschlußplatten 5 und 6 eine übliche Größe haben, die im Interesse geringer Druckkräfte zwischen den Anschlußplatten und den zugehörigen Wirbelkörpern bemessen ist, hat der Überbrückungsteil 7 eine Breite, die geringer ist als diejenige des zugehörigen Wirbelkörpers 3, nämlich so gering, daß der Überbrückungsteil in eine von der Vorderseite in den betreffenden Wirbelkörper 3 eingearbeitete Ausnehmung eingesetzt werden kann und die daneben verbleibende Knochensubstanz zur sicheren Verankerung des Überbrückungsteils in der Ausnehmung ausreicht.

Zumindest ein Teil der Ausnehmung hat eine Gestalt, die möglichst genau mit der Gestalt des Überbrückungsteils 7 übereinstimmt. Dadurch wird eine im wesentlichen spielfreie Anlage der Implantatoberfläche an der künstlich geschaffenen Oberfläche des Knochens möglich. Zum einen ergibt dies eine gute gegenseitige Abstützung. Zum anderen ergibt sich daraus die Möglichkeit einer durch Knochenwachstum entstehenden stabilen Verbindung zwischen dem Knochen und dem Implantat. Und schließlich gibt dies Sicherheit dafür, daß die am Implantat vorgesehenen Verankerungsvorsprünge 9 mit im wesentlichen ihrer vollen Länge in das Knochengewebe eingreifen, um die Verankerungskräfte übertragen zu können.

Das Ziel der Formübereinstimmung zwischen dem Überbrückungsteil und der im Knochen geschaffenen Ausnehmung erreicht man am leichtesten mit einer Trapezform des Querschnitts des Überbrückungsteils, wie sie in Fig. 3 angedeutet ist. Es können auch andere Querschnittsformen verwendet werden, vorzugsweise aber solche, die sich von vorne nach hinten verjüngen, damit beim Einsetzen des Überbrückungsteils in die Ausnehmung die erwähnte spielfreie Anlage der Seitenflächen an den Resektionsflächen des Knochens zustandekommt.

Das Merkmal, daß der Überbrückungsteil eine nach hinten schmaler werdende, insbesondere trapezförmige Querschnittsform hat, soll ggf. Schutz unabhängig von den kennzeichnenden Merkmalen des Anspruchs 1 genießen.

Die Verankerungsvorsprünge sind starr an den Seitenflächen 10 des Überbrückungsteils 7 angeordnet. Gemäß Fig. 3 haben sie die Form einer Vielzahl von kleinen, spitzen Erhebungen 11, die beim Einschieben des Implantats in die Ausnehmung des Wirbels sich in die Knochenoberfläche eindrücken. In einer nicht dargestellten alternativen Ausführung sind sie größer und klingenförmig ausgebildet, wobei die Klingenebene in Einschubrichtung verläuft, so daß sie selbstschneidend in die Knochensubstanz eindringen. Eine wieder andere Ausführungsform verwendet Mikrovorsprünge in Gestalt einer Rauhigkeit, welche die gesamte Seitenfläche 10 oder einen wesentlichen Teil derselben überzieht. In jedem Fall können zusätzlich zu den Verankerungsvorsprüngen Öffnungen 12 oder Poren vorgesehen sein, in denen hineinwachsende Knochensubstanz sich verankern kann. Um diesen Prozeß zu beschleunigen, können die Öffnungen vorab mit Knochenspänen gefüllt werden. Auch eine Beschichtung des Implantats mit osteokonduktiver oder osteoinduktiver Substanz ist möglich.

## Patentansprüche

1. Prothese zum Teilersatz eines Wirbelkörpers mit einer oberen Anschlußplatte (5) zur Verbindung mit einem oberen Wirbelkörper (1), einer unteren Anschlußplatte (6) zur Verbindung mit einem unteren Wirbelkörper (2) und einem die obere und die untere Anschlußplatte (5, 6) verbindenden Überbrückungsteil (7), der zum Überbrücken mindestens eines zwischen dem oberen und dem unteren Wirbelkörper (1, 2) befindlichen und teilweise zu ersetzenden Wirbelkörpers (3) ausgebildet ist und mit Einrichtungen zur Befestigung an diesem ausgerüstet ist, **dadurch gekennzeichnet, daß** der Überbrückungsteil (7) zur Befestigung am Wirbelkörper (3) seitlich vorragende, starre Verankerungsvorsprünge (9, 11, 15, 32) aufweist.

2. Wirbelsäulenprothese nach Anspruch 1, **dadurch gekennzeichnet, daß** die Querschnittsform des Überbrückungsteils (7) rechteckig oder nach hinten schmaler werdend ist.

3. Wirbelsäulenprothese nach Anspruch 1, **dadurch gekennzeichnet, daß** die Verankerungsvorsprünge als Rauheit ausgebildet sind.

4. Wirbelsäulenprothese nach Anspruch 1, **dadurch gekennzeichnet, daß** der Überbrückungsteil (7) Öffnungen (12) oder Poren für die Aufnahme von Knochengewebe aufweist.

5. Wirbelsäulenprothese nach Anspruch 4, **dadurch gekennzeichnet, daß** sie mit einer Füllung von Knochenmaterial oder Knochenersatzmaterial versehen ist.
